# EUROPEAN PATENT APPLICATION

(11) **EP 2 356 916 A1**
(43) Date of publication of application: **17.08.2011**
(21) Application number: 09828660.2
(22) Date of filing: 23.11.2009
(51) Int. Cl.: A43B 17/02, A61F 5/14

(54) **FLEXIBLE PRESSURE SHEET**

(30) Priority: 27.11.2008 ES 200803382; 13.11.2009 ES 200902168
(71) Applicant: Chasco Pérez de Arenaza, Juan Carlos, E-31010 Pamplona (Navarra) (ES)
(72) Inventor: Chasco Pérez de Arenaza, Juan Carlos, E-31010 Pamplona (Navarra) (ES)
(74) Representative: Torner Lasalle, Elisabet
(86) International application number: PCT/ES2009/000540
(87) International publication number: WO 2010/061015

(57) **Abstract**

The invention relates to a flexible sheet which includes cavities intercommunicated by a captive fluid of a known viscosity and which is positioned as an insole beneath the sole of the foot. The upper surface of the aforementioned cavities receives plantar pressures, thereby acquiring a corresponding dimpled appearance indicating those areas receiving greater or less plantar pressure. The gradual movement of the fluid through the hollow closed circuit is caused mainly by the resistance to the fluid provided by two-way an one-way valves and alternatively by the resistance provided by the cross-section and length of the conduits to the viscosity of the fluid. In this way, as the foot oscillates the cavities remain the same. The invention is mainly intended for obtaining approximate plantar pressure measurements by observing the upper surface of the adjoining cavities after a sufficient number of steps, producing the mould or negative and saving a record of same, and producing the final insole.

## Description

### Object of the invention

As explained in this descriptive specification, the current invention relates to the improvement in the design of a flexible sheet described in patent P200601693 ES, mechanically and visually assessing pressures; making subsequent moulds with it and the subsequent use mostly for medical purposes, which can mitigate any risks to the skin of its wearer.

### Background of the invention

Quite often, the sole of the foot presents alterations, mostly produced by excessive, repetitive pressures in some parts of the said sole. There are many devices available in the market that detects both static and dynamic pressures; some are mechanical and others, electronic devices informing the specialist about the footfall. These pieces of knowledge pose problems that must be subsequently solved by the foot specialist, who usually has limited time and resources. By means of an insole that can be easily placed in a shoe, sneaker or slipper, the device of my invention provides, through a fast, simple, mechanical procedure, firstly, a view of how the footfall of a subject under study is created and, after a number of cycles of walking or stepping, in the footstep left, we shall see a number of valleys, corresponding to areas of maximum repeated impacts, and a number of high areas, wherein the foot is not supported or hardly is. A qualified technician in the art can obtain, with a quick glance, the view of a good approximation to the average of the dynamic plantar pressures in the different areas of the sole of the foot for each person measured; the sheet being susceptible to being automatically reused later again. From this footstep a negative cast can easily be obtained, high areas in this negative corresponding to areas of maximum impact and very thin areas corresponding to areas of low or zero support. The material used for the negative cast is normally of low hardness and excellent memory. With methods used frequently in the market (by chiropodists, orthopaedists, prosthetists, etc.), for instance of silicone; this negative cast can be kept in the medical record of the subject under study.

Another possibility for this sheet with cavities is, in many cases, the generation of the final insole that the subject will later use to compensate for the excessive pressures; this way, covering the negative cast up to its highest parts, which, as indicated, corresponds to the areas of greatest plantar impact, with a harder material, this compound sheet (soft plus hard) can be used in many subjects under study as a final insole and, in some cases, at least do as a final insole for its metatarsal areas.

Areas without support or with little support will raise more and this will cause the sole of the foot to enjoy greater surface support, lift and stability. On the other hand, in the areas of maximum impact, the latter will have decreased and the soft sheet with excellent memory will absorb these impacts. The present patent description is disclosed in the patent applications P200803382 and P200902168.

### Disclosure of the invention

The device of my invention is a sheet (1); on top of it there are a number of cavities (2) adjoined or close to one another and linked together my means of a captive circuit through which a fluid of known density circulates. This hollow and closed circuit with the circulating fluid is usually in the lower portion of the said sheet or the adjoining cavities.

The slow transfer of fluid between the cavities occurs in a significant manner: Through the resistance presented by the passageway surface of the holes and ducts, as well as the length of the said ducts, which can be tubes, channels, sieves or any grooves (3) acting as a brake to the viscosity of the fluid.

Cavities receive the pressures (6) of the sole of the foot from their upper side. When, in one of the cavities, a predetermined pressure is exceeded, its fluid is transferred between adjacent cavities, increasing or decreasing the volume of the said cavities in inverse proportion to the pressures experienced by the different cavities from their upper side. The deformation of the volume of the different cavities is mostly in the vertical direction, both upwards and downwards. During the footfall, the fluid is transferred from some cavities to adjacent ones; when the foot sways again, the heights and the volume of the cavities become stable; that way, cavities that have received strong pressures have had their volume reduced, and the height of adjacent cavities wherein the pressure has been smaller or become zero will be increased, since they have received a part of the transferred fluid. This situation of change remains stable during the swaying of the foot, until new pressures appear that are sufficient to overcome the internal resistance described to the passage of the viscous fluid, altering the said situation of equilibrium. Repeating this succession of pressures a sufficient number of steps will allow the flexible sheet to give us a good approximation to the average of pressures in each of the areas; for example, of the sole of a foot after walking, since not always, under similar conditions, is the footprint the same in the different areas of the foot for one person.

The cavities with the holes, channels, sieves, tubes or grooves are occupied in captive form by a fluid, which contacts and occupies all that volume and from which some volume has previously been subtracted after drawing in the air and part of the fluid, achieving it thanks to the flexibility of the cavities; this allows any chamber that barely receives pressure to rise more than in its original situation of equilibrium, so the areas that did not support or press get closer to the sole of the foot, thereby giving stability and support to the insole to be developed later.

The objective is to obtain a plantar footstep assessable to the naked eye that is the average of the pressures produced by the footfall of a person's foot, after walking with the flexible sheet for some time, and then to be able to reuse it automatically on another person.

The mould or negative cast can be obtained directly from the said footprint produced by each person. For instance, we add liquid silicone and wait for its quick setting. We separate the generated soft foil from the flexible sheet, with which we have the negative cast, which we can keep in the patient's medical record.

Later on, we can directly produce the complete insole from the said mould. This way, the chiropodist or orthopaedist can work on this negative cast leaving, removing or adding material. Lastly, this soft foil can be covered by a harder one, thereby obtaining the final sheet. In general, the areas of the foot with greater impact will have a softer area in the insole so as to absorb impacts, and the areas whose pressure or support was low or zero will have moved closer to the sole of the foot so as to increase their support and area of support in these areas of the final, harder insole, with subsequent adaptations of the plantar arches.

### Description of the drawings

For better understanding what is described in the current specification, several drawings are attached that, only by way of example, represent a number of embodiments for carrying out the flexible pressure sheet.

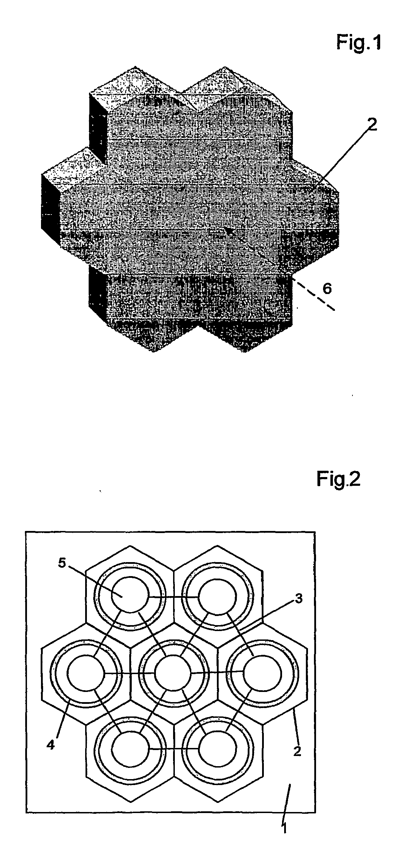

Figure 1. A flexible sheet of contiguous or adjoining hexagonal cavities (1), seeing their upper convexity, wherein it receives the different plantar pressures or impacts, in perspective. In this example the cavities have a hexagonal shape and they are joined together by their base.
Figure 2 Sheet (1), situated under the cavities it supports, this support being fostered by the circular groove (4) found on the upper side of the said sheet. Holes (5) connecting the ducts (3) below this sheet with the inlet to the upper cavities. Ducts linking adjacent holes and carrying the fluid.

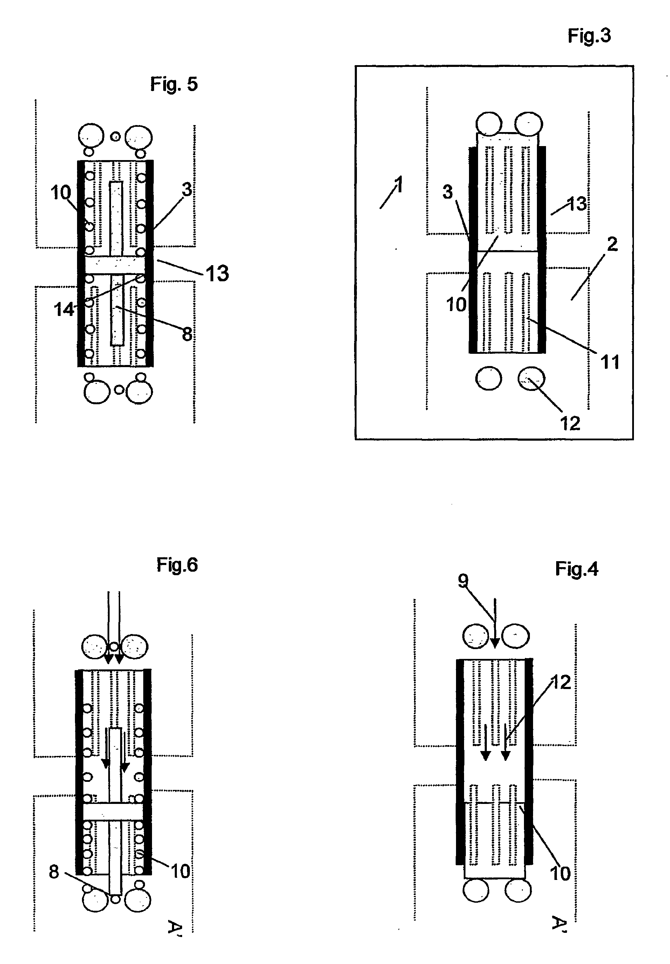

Figure 3. A longitudinal sectional view of a tube (3) communicating two contiguous cavities (2) that forms a valve device (4), supported on a basal sheet (1), here with an elastomeric spring (10) and a number of caps (7) located at both ends of each tube with a number of channels in its inner wall (11).
Figure 4. A longitudinal sectional view of a tube communicating two contiguous cavities that forms a valve device, here with an elastomeric spring contracted (10) by the action of the internal pressure (9) of the fluid (12) filling it, in one of the directions, by increasing the pressure in one of the adjacent cavities.
Figure 5. A longitudinal sectional view of a tube (3) communicating two contiguous cavities that forms a valve device (13), here with a metal spring (10) with its spring rod (8) and a spring limit (14) closing with its outside boundary, in contact with the internal boundary of the tube; the flow of the fluid is normally shut off until, due to the intensity of the pressure, this spring limit surpasses the channels.
Figure 6. A longitudinal sectional view of a tube communicating two contiguous cavities that forms a valve device, here with a contracted metal spring (10) with its rod (8) seated on its cap.

### Description of the preferred invention

The device of my invention is a flexible sheet formed by cavities, sheets, tubes and a captive fluid. Cavities (2) adjoined to one another are supported on a sheet (1) with grooves (4) on its upper side and holes (5), the latter linking the upper cavities with the ducts arranged under the sheet (4). The cavities and the channels (3) linking them are occupied by a captive fluid of known viscosity. These cavities receive the pressures (6) of the sole of the foot on their upper side. When in one of the cavities a certain pressure is exceeded, its fluid is transferred between adjacent cavities, thereby increasing or decreasing the volume of the said cavities in inverse proportion to the pressures experienced by the different cavities from their upper side. The deformation of the volume of the different cavities is mostly in the vertical direction, both upwards and downwards. The ducts are generally located under the cavities, their endpoints coinciding under the inlet hole (5) of the said cavities. The ducts, in turn, are located on a laminated polymer or a sole (7), below.

The ducts are made of a material with sufficient strength to withstand intracavity pressures, generally of polymeric materials.

Each duct links a cavity with a contiguous cavity, several ducts coming together under one and the same inlet hole to each cavity and they are usually separated within the hollow of the inlet.

During the footfall, the fluid is transferred from some cavities to adjacent ones; when the foot sways again, the heights of the cavities become stable; that way, cavities that have received strong pressures have had their volume reduced, and the height of adjacent cavities wherein the pressure has been reduced or become zero will be increased, since they have received a part of the transferred fluid. This situation of change remains stable during the swaying of the foot, due, to a large extent, to the resistance offered to the circulating fluid by the ducts, until new pressures appear that are sufficient to alter the said situation of equilibrium. With the repetition of this succession of pressures a sufficient number of steps, the flexible sheet will give us a good approximation to the average of pressures in each of the areas; for example, of the sole of a foot after walking, since not always, under similar conditions, is the footprint the same for one person.

The objective is to obtain a plantar footstep assessable to the naked eye that is the average of the pressures produced by the footfall of a person's foot, after walking with the flexible sheet for some time, and then to be able to reuse it automatically on another person.

A mould or negative cast can be obtained directly from the said footprint produced; the entire insole can even be developed directly from the said mould; on other occasions, the final insole at least of the metatarsal area is obtainable, with subsequent adaptations in the area of the heel, the toes and the plantar arches.

The cavities with the holes and ducts are occupied in captive form by a fluid, which contacts and occupies all that volume and from which some volume has previously been subtracted after drawing in the air and part of the fluid, achieving it thanks to the flexibility of the cavities; this allows any chamber that barely receives pressure to rise more than in its original situation of equilibrium, so the areas that did not support or press get closer to the sole of the foot, thereby giving stability and support to the insole to be developed later.

## Claims

1. The device of my invention is a sheet formed by adjoining cavities linked with each other through a hollow and closed circuit whose interior is occupied by a fluid of known viscosity, as described in patents P200601693, P200803382 and P200902168. It is **characterised in that**: A) The resistance to the flow of viscous fluid through the hollow circuit between cavities allows its slow flow between the said cavities. B) This resistance allows the said cavities to remain in equilibrium during the swaying of the foot in walking. C) A sufficient number of cycles or steps of normal walking provides the upper side of each adjoining cavity a larger or smaller height in inverse proportion to the plantar pressures received. D) We can obtain a negative mould of the upper side of the adjoining cavities and preserve it; from this mould we obtain the final corrective full insole or, at least, one of the metatarsal area.

2. According to 1. The linked cavities have a number of ducts or tubes with bidirectional valve members.

3. According to 1. The linked cavities have a number of dual ducts or tubes with one-way valve members.

4. According to 1. The hollow and closed circuit linking the cavities is endowed with a number of ducts that, due to their diameter or length, present resistance in a preestablished way to the viscous fluid.

5. According to 4. The hollow and closed circuit is formed by tubes.

6. According to 4. The hollow and closed circuit is formed by ducts with tubes.

7. According to 4. The hollow and closed circuit is formed by sieves.

8. According to 4. The hollow and closed circuit is formed by grooves.
